# EUROPEAN PATENT APPLICATION

(11) **EP 3 875 084 A1**
(43) Date of publication of application: **08.09.2021**
(21) Application number: 19880084.9
(22) Date of filing: 29.10.2019
(51) Int. Cl.: A61K 31/353, A61P 1/00, A61P 31/04, A61P 35/00

(54) **ANTIBACTERIAL AGENT AGAINST ENTEROBACTERIUM WHICH CONTAINS EQUOL AS ACTIVE INGREDIENT**

(30) Priority: 29.10.2018 JP 2018203055
(71) Applicant: Tateda, Kazuhiro, Kanagawa 236-0057 (JP); Kimura, Soichiro, Tokyo 143-8540 (JP); Tanaka, Yumi, Hiroshima 739-1743 (JP)
(72) Inventor: Tateda, Kazuhiro, Kanagawa 236-0057 (JP); Kimura, Soichiro, Tokyo 143-8540 (JP); Tanaka, Yumi, Hiroshima 739-1743 (JP)
(74) Representative: Hoffmann Eitle
(86) International application number: PCT/JP2019/042299
(87) International publication number: WO 2020/090789

(57) **Abstract**

Provided is an antibacterial drug for an enterobacterium that causes an infection in humans, such as *Clostridioides difficile.* An antibacterial agent against an enterobacterium, comprising equol as an active ingredient.

## Description

### Technical Field

The present invention relates to an antibacterial agent against enterobacteria, in particular *Clostridioides difficile.*

### Background Art

*Clostridioides difficile* (*C*. *difficile*) is a causal bacterium for *Clostridioides difficile* infection (CDI), which presents as diarrhea and abdominal pain as symptoms and sometimes leads to death. *Clostridioides difficile* infection is a problematic nosocomial infection (see Non Patent Literature 1).

Antiprotozoal drugs such as metronidazole and antibiotics such as vancomycin are used as an antibacterial drug against *Clostridioides difficile* (*C. difficile*) infection, but once *Clostridioides difficile* forms spores, effects of these antibacterial drugs are disadvantageously decreased.

*Clostridioides perfringens* (*C. perfringens*) is an indigenous bacterium in humans, and also detected from domestic animals and fishes. *Clostridioides perfringens* is resistant to heat, and known to be a causal bacterium for food poisoning.

*Bacteroides fragilis* (*B. fragilis*) is an indigenous bacterium in humans, and there exists a toxic type of the bacterium. Moreover, *Bacteroides fragilis* is said to be a cause for colorectal cancer.

Equol is a final metabolite in soybeans, and biosynthesized from soy isoflavone by enterobacteria. Equol has a structure similar to that of estrogen, a female hormone, and hence a method for producing soybean-derived equol has been established (see Patent Literature 1). Equol is used as an active ingredient of supplements useful for reinforcement of bones and so on (see Patent Literature 2).

### Citation List

### Patent Literature

Patent Literature 1: JP Patent Publication (Kokai) No. 2017-18120A
Patent Literature 2: JP Patent Publication (Kokai) No. 2013-184959A

### Non Patent Literature

Non Patent Literature 1: Shigeru KAMIYA, MODERN MEDIA, vol. 56 No. 102010 [A Topical Infection] pp. 233-241

### Summary of Invention

### Technical Problem

An object of the present invention is to provide an antibacterial drug for an enterobacterium that causes an infection in humans, such as *Clostridioides difficile.*

### Solution to Problem

The present inventors examined effects of equol synthesized in the intestine of humans on enterobacteria. The results confirmed that equol inhibits the growth of enterobacteria such as *Clostridioides difficile* in a concentration-dependent manner and further inhibits the sporulation of *Clostridioides difficile,* and the present inventors found that equol can be used as an antibacterial agent for enterobacteria such as *Clostridioides difficile,* thus completing the present invention.

Specifically, the present invention is as follows.
[1] An antibacterial agent against an enterobacterium, comprising equol as an active ingredient.
[2] The antibacterial agent according to [1], wherein the enterobacterium is selected from the group consisting of *Clostridioides difficile, Clostridioides perfringens* (*C. perfringens*), and *Bacteroides fragilis* (*B. fragilis*).
[3] The antibacterial agent according to [1] or [2], wherein the antibacterial agent inhibits the growth of *Clostridioides difficile* and inhibits the sporulation of *Clostridioides difficile.*
[4] A preventive or therapeutic agent for *Clostridioides difficile* infection, comprising the antibacterial agent according to any one of [1] to [3].
[5] A drug for controlling intestinal function comprising equol as an active ingredient.
[6] The drug for controlling intestinal function according to [5], wherein the drug for controlling intestinal function mitigates symptoms or a symptom of diarrhea and/or abdominal pain caused by the enterobacterium.
[7] A preventive and/or therapeutic agent for colorectal cancer, comprising equol as an active ingredient.

The present specification includes contents disclosed in JP Patent Application No. 2018-203055, to which the present application claims priority.

### Advantageous Effects of Invention

*Clostridioides difficile* exhibits resistance to antibacterial drugs through formation of spores, and causes an infection by secreting a toxin. Moreover, Clostridioides difficile causes nosocomial infection. Equol is capable of inhibiting the growth of *Clostridioides difficile* in a concentration-dependent manner and inhibiting the sporulation of *Clostridioides difficile,* and hence can be used as an antibacterial drug for *Clostridioides difficile,* and can further prevent nosocomial infection due to *Clostridioides difficile.*

### Brief Description of Drawings

[Figure 1] Figure 1 shows a graph demonstrating the growth inhibitory effect of equol on a *Clostridioides difficile* 027 strain.
[Figure 2] Figure 2 shows a graph demonstrating the influence of soy isoflavone on the growth of *Clostridioides difficile.*
[Figure 3] Figure 3 shows graphs demonstrating the growth inhibitory effect of equol on four bacterial strains of *Clostridioides difficile.*
[Figure 4] Figure 4 shows graphs demonstrating the growth inhibitory effect of equol on other bacterial species.
[Figure 5] Figure 5 shows photographs demonstrating the influence of equol on the form of *Clostridioides difficile.*
[Figure 6] Figure 6 shows a graph demonstrating the sporulation inhibitory effect of equol on *Clostridioides difficile.*

### Description of Embodiments

Hereinafter, the present invention will be described in detail.

The present invention is an antibacterial agent against an enterobacterium, comprising equol (4',7-isoflavandiol) as an active ingredient.

In the present invention, "antibacterial" refers not to killing bacteria, but to suppressing or inhibiting the growth and proliferation of bacteria, and thus differs from "bactericidal" and "sterilizing".

Equol is a final metabolite in soybeans, and biosynthesized from soy isoflavone by enterobacteria. The chemical formula of equol is C₁₅H₁₄O₃, and the structural formula is shown in the following.

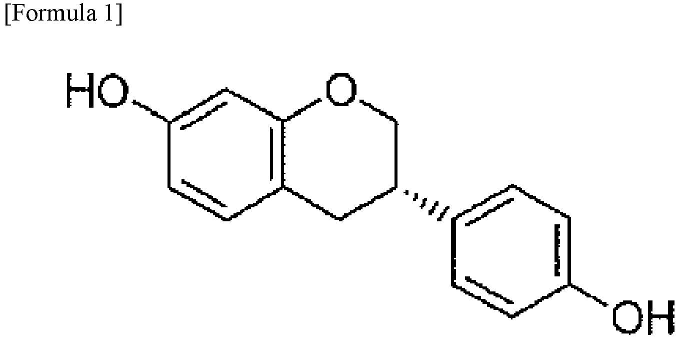

Some human individuals are capable of synthesizing equol in the intestine and others are not, and the capability depends on the presence or absence of equol-producing bacteria in his or her intestinal bacterial flora.

Equol has antibacterial activity against some enterobacteria. Examples of enterobacteria for which equol has antibacterial activity include *Clostridioides difficile* (*C. difficile,* CD), *Clostridioides perfringens* (*C. perfringens* (*Clostridium perfringens*)), and *Bacteroides fragilis* (*B. fragilis*), and equol is effective for *Clostridioides difficile* among them.

*Clostridioides difficile* is known to cause *Clostridioides difficile* infection (CDI), a representative nosocomial infection. *Clostridioides difficile* infection is a gastrointestinal infection which causes enteritis together with symptoms of diarrhea and abdominal pain, and involves fever or leukocytosis in some cases, occasionally leading to death in serious cases. *Clostridioides difficile* forms spores, and if *Clostridioides difficile* or spores thereof are contained in feces from a CDI patient, in particular, if spores of *Clostridioides difficile* are contained therein, the feces may allow *Clostridioides difficile* to remain in a hospital environment including toilet facilities, beds, and floors to cause a serious problem of nosocomial infection. Antiprotozoal drugs such as metronidazole and antibiotics such as vancomycin are used as an antibacterial drug against *Clostridioides difficile,* but once forming spores, *Clostridioides difficile* exhibits resistance to antibacterial drugs, and effects of these antibacterial drugs are disadvantageously decreased. Remaining spores germinate, resulting in the recurrence of *Clostridioides difficile* infection.

*Clostridioides perfringens* (*C. perfringens* (*Clostridium perfringens*)), also called Welch bacillus, is present in the intestine of animals including humans, domestic animals, and fishes, and releases the toxin enterotoxin to cause food poisoning. Since being widely distributed in soils, rivers, oceans, and so on, *Clostridioides perfringens* often contaminates foods and cooking utensils, and the spore form thereof frequently induces food poisoning because of the thermal durability. In addition, *Clostridioides perfringens* has been reported to be involved in aging, cancer, and so on.

*Bacteroides fragilis* (*B. fragilis*) is an obligate anaerobic Gram-negative bacillus present in the intestine of humans, and does not form a spore. *Bacteroides fragilis* may cause an infection when it intrudes into blood vessels or the abdominal cavity. *Bacteroides fragilis* has been reported to be involved in the onset of colorectal cancer.

Equol inhibits the growth of the above-described enterobacteria, in particular, *Clostridioides difficile* in a concentration-dependent manner, and further inhibits the sporulation thereof. Equol can ameliorate and prevent *Clostridioides difficile* infection by inhibiting the growth of *Clostridioides difficile* in the intestine. By inhibiting the growth of *Clostridioides difficile* in the intestine, equol can prevent *Clostridioides difficile* infection from propagating through nosocomial infection or the like. Moreover, equol is capable of inhibiting the sporulation of *Clostridioides difficile* in the intestine, and hence can prevent *Clostridioides difficile* infection from propagating via spores.

Equol is useful for subjects without equol-producing bacteria in the intestinal bacterial flora and subjects with equol-producing bacteria in the intestinal bacterial flora.

Whether equol has antibacterial activity against an enterobacterium or not can be examined by culturing the enterobacterium to reach a certain bacterial cell count, adding equol in different concentrations to the medium, and subsequently culturing to check the influence on the growth. Culture can be suitably performed, for example, under anaerobic conditions at 30 to 40°C.

Equol is synthesized from daidzin contained in soybeans as a starting material through a pathway of daidzin → daidzein → dihydrodaidzein → equol by the actions of enterobacteria. Equol can be synthesized by using bacteria involved in these reactions or enzymes extracted from such bacteria. Methods for producing equol are described in JP Patent Publication (Kokai) No. 2017-205117A, JP Patent Publication (Kokai) No. 2017-18120A, JP Patent Publication (Kokai) No. 2013-188220A, WO2009/084603, and so on, and equol can be produced in accordance with any of these descriptions.

Equol may be eighter a synthesized product or a product obtained by fermentation. Any material containing daidzein may be used as a material to ferment for a product obtained by fermentation, and the material is, for example, that derived from soybeans and/or soybean hypocotyls. Examples of equol include equol in fermented products such as equol-containing fermented products of soybean hypocotyls, equol-containing fermented products of soybean hypocotyl extract, equol-containing fermented products of soybeans, and equol-containing fermented products of soybean extract.

The antibacterial agent against an enterobacterium, comprising equol as an active ingredient according to the present invention can prevent propagation of pollution with *Clostridioides difficile, Clostridioides perfringens,* or *Bacteroides fragilis* when being applied to facilities and instruments in hospitals, and against *Clostridioides difficile,* for example, can further prevent the propagation of *Clostridioides difficile* infection by inhibiting the growth. In this case, equol can be formulated for use into a dosage form, such as a water-dispersible powder, a liquid, an oil solution, a dusting powder, a grain, a sol (a flowable agent), and a gel, containing 10 µg/mL to 100 g/mL of equol.

The antibacterial agent against an enterobacterium, comprising equol as an active ingredient according to the present invention can be used as a pharmaceutical composition to administer to a subject infected with *Clostridioides difficile, Clostridioides perfringens,* or *Bacteroides fragilis,* and, for example, is a preventive or therapeutic agent for *Clostridioides difficile* infection.

The pharmaceutical composition comprising equol as an active ingredient can be used as a drug for controlling intestinal function. The drug for controlling intestinal function can be used to mitigate symptoms or a symptom of diarrhea and/or abdominal pain caused by an enterobacterium.

Further, enterobacteria for which equol has antibacterial activity are known to be associated with the onset of colorectal cancer. Accordingly, the pharmaceutical composition comprising equol as an active ingredient can be used as a preventive and/or therapeutic agent for colorectal cancer.

The antibacterial agent and preventive or therapeutic agent may contain a physiologically acceptable carrier or diluting agent. Examples of the carrier include physiological saline, phosphate-buffered saline, glucose solution, and buffered physiological saline. The pharmaceutical composition for prevention or treatment of infections to be used for subjects infected with *Clostridioides difficile, Clostridioides perfringens,* or *Bacteroides fragilis* can be administered in various forms. For example, the pharmaceutical composition can be administered through oral administration in the form of a tablet, a capsule, a granule, a powder, or a syrup, or through parenteral administration in the form of an injection, an infusion, or a suppository. Although the dose depends on symptoms, age, body weight, and so on, the dose in oral administration for adults is typically approximately 0.01 mg to 1000 mg per day, and this can be provided in one administration or separately in several administrations. In the case of parenteral administration, a dose of approximately 0.01 mg to 1000 mg can be suitably provided in each administration by subcutaneous injection, intramuscular injection, or intravenous injection.

The present invention includes food compositions containing equol. Examples of such food compositions include confectioneries (e.g., cookies, biscuits, chocolates, chips, cakes, chewing gums, candies, gummy candies, manju (sweet buns), yokan (sweet bean jellies), puddings, jellies, yogurt, ice creams, sherbets), breads, noodles, rice foods, cereal foods, beverages (e.g., liquids, soft drinks, carbonated beverages, nutritional beverages, powdered beverages, fruit beverages, milk beverages, jelly beverages), soups (powdered, freeze-dried), and miso (fermented soybean paste) soups (powdered, freeze-dried).

The food composition encompasses not only normal foods but also functional foods, for example, including health foods, foods with functional claims, foods for specified health uses, and foods with nutrient function claims. Foods for specified health uses are foods that are ingested for a specific health purpose in meals and shows the indication that the health purpose is expected to be achieved by the ingestion. These foods may be provided, for example, with the indication that this is used for controlling intestinal function or that this is used for mitigation of diarrhea or abdominal pain.

With the food composition according to the present invention, for example, preferably 0.1 mg to 30 mg, more preferably 2 mg to 20 mg, even more preferably 3 mg to 10 mg of equol can be provided per ingestion. Examples of the lower limit value of the amount of equol per ingestion include 0.2 mg, 0.5 mg, 1 mg, 2 mg, 3 mg, 4 mg, and 5 mg, examples of the upper limit value include 30 mg, 20 mg, 10 mg, 6 mg, and 5 mg, and a preferred range of the amount of equol per ingestion can be represented by a combination of the upper limit value and the lower limit value.

As mentioned above, it is preferable that the amount of equol ingested per day with the food composition according to the present invention be 30 mg or less. The amount of equol ingested per day is preferably 0.1 mg to 30 mg, more preferably 2 mg to 20 mg, and even more preferably 3 mg to 10 mg. Examples of the lower limit value of the amount of equol ingested per day include 0.2 mg, 0.5 mg, 1 mg, 2 mg, 3 mg, 4 mg, and 5 mg, examples of the upper limit value include 30 mg, 20 mg, 10 mg, 6 mg, and 5 mg, and a preferred range of the amount of equol ingestible per day can be represented by a combination of the upper limit value and the lower limit value. Equol in an amount ingestible per day may be provided in one ingestion, or provided separately in multiple ingestions (e.g., two, three, four, and five ingestions).

### Examples

The present invention will be specifically described with reference to Examples below; however, the present invention is not limited to these Examples.

### [Example 1] Growth Inhibition by Equol on Clostridioides difficile (C. difficile) 027 Strain

*Clostridioides difficile* (NAP1/027 strain) was added to a medium containing equol in a concentration of 100 µg/mL, 75 µg/mL, 50 µg/mL, or 25 µg/mL (0 hour), and subsequently cultured to check the influence on the growth for 24 hours after the start of culture. The culture performed was totally under anaerobic conditions at 35°C.

Because equol dissolved in DMSO was used, a medium with addition only of DMSO was additionally subjected to measurement as a control.

The growth of the bacterium was examined by measuring the absorbance of each medium at 600 nm (OD600).

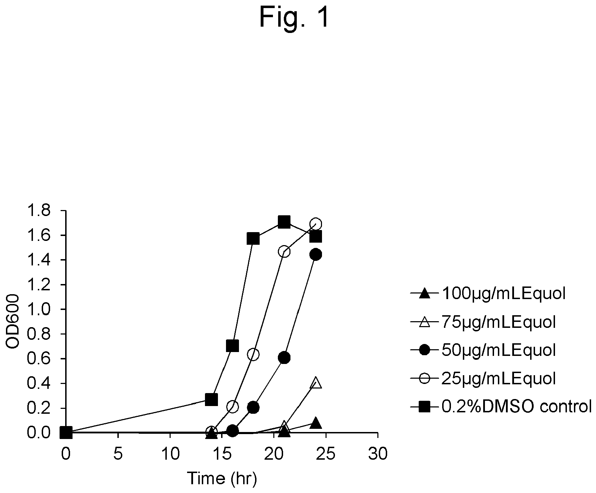

Figure 1 shows the results. As demonstrated in Figure 1, the growth of *Clostridioides difficile* in each of the media with addition of equol was slower than that in the control, which suggested inhibition of growth by equol. The growth rate of *Clostridioides difficile* was lower as the concentration of equol added was higher, and thus the inhibition depended on the concentration of equol.

### [Example 2] Influence of Soy Isoflavone on Growth of Clostridioides difficile (NAP1/027 Strain).

Equol is a final product generated from daidzin through daidzein and dihydrodaidzein. Inhibition of the growth of *Clostridioides difficile* by equol (100 µg/mL, 50 µg/mL), daidzein (50 µg/mL), and dihydrodaidzein (100 µg/mL, 50 µg/mL) was measured in the same manner as in Example 1.

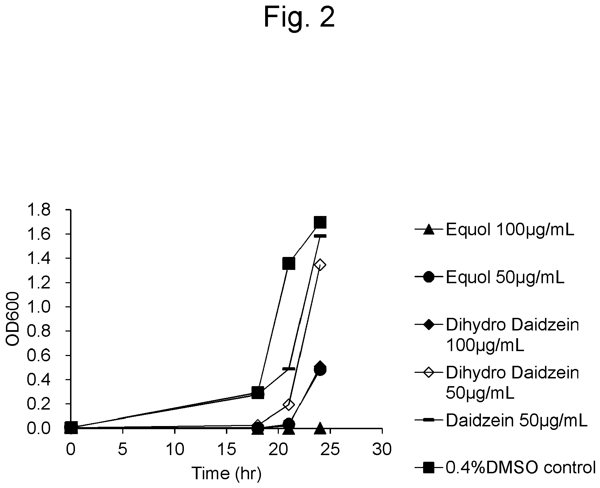

Figure 2 shows the results. As demonstrated in Figure 2, although no clear difference from the control was found in the media with addition of 50 µg/mL of daidzein or dihydrodaidzein, the medium with 100 µg/mL of dihydrodaidzein and the medium with 50 µg/mL of equol inhibited the growth of *Clostridioides difficile* to the same degree, and the medium with 100 µg/mL of equol exhibited an even higher degree of inhibition than them.

### [Example 3] Growth Inhibition on Four Bacterial Strains of Clostridioides difficile

Whether clinical isolates of *Clostridioides difficile* other than the 027 strain (TUM12745 strain, TUM12806 strain, and TUM12855 strain) also undergo growth inhibition by equol (100 µg/mL) was validated in the same manner as in Example 1.

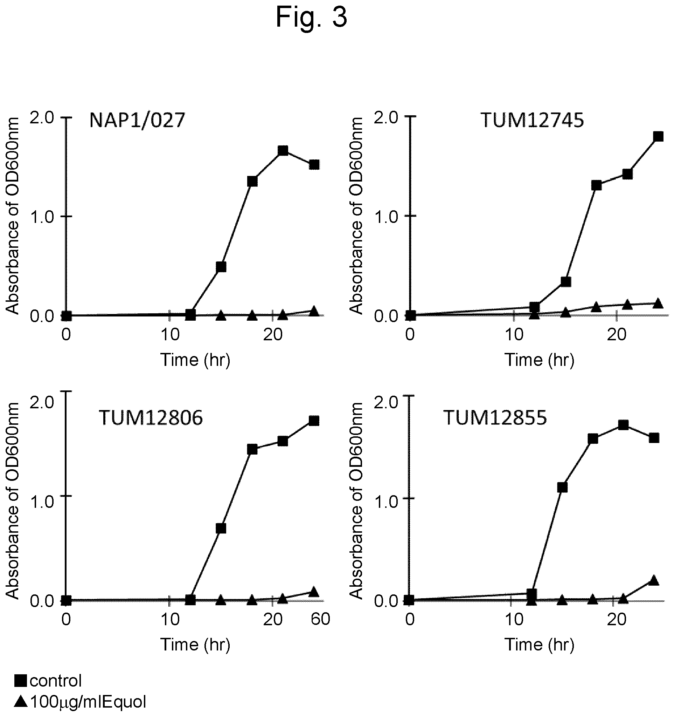

Figure 3 shows the results. As demonstrated in Figure 3, similar inhibition was found for all the bacterial strains used in the experiment.

### [Example 4] Growth Inhibition on Other Bacterial Species

Whether equol (100 µg/mL) exhibits inhibition against bacteria other than *Clostridioides difficile* was validated in the same manner as in Example 1.

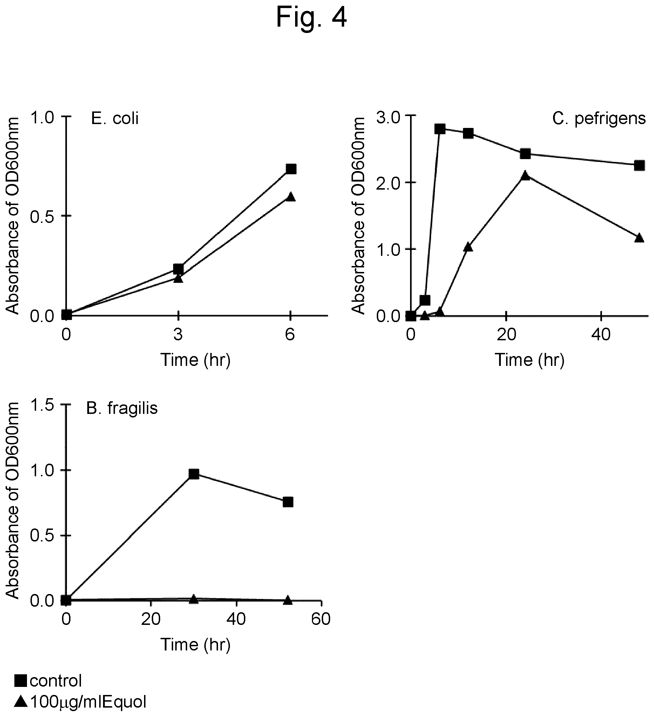

Figure 4 shows the results. As demonstrated in Figure 4, no influence was found for Escherichia coli (E. coli). An influence was observed for *Clostridioides perfringens* (*C. perfringens* (*Clostridium perfringens*)) and *Bacteroides fragilis (B. fragilis).* In particular, completely no proliferation was found for *Bacteroides fragilis,* and when being transferred into a medium without equol at the end of 52 hours of the test, *Bacteroides fragilis* formed a colony; this revealed that only the growth was inhibited, without killing the bacterium.

### [Example 5] Influence on Form (Gram Staining)

The influence of equol on the form of *Clostridioides difficile* (NAP1/027 strain) was validated through staining of *Clostridioides difficile* with Gram staining under the action of equol (100 µg/mL) in the same manner as in Example 1.

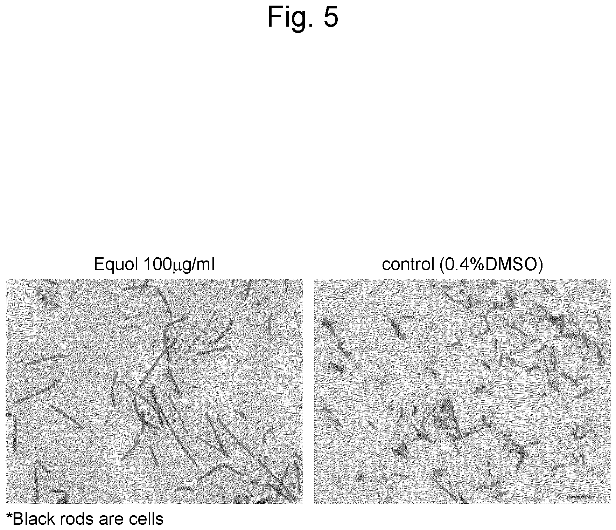

Figure 5 shows the stained images. Figure 5 shows images of Gram staining after 8-day culture. Cells with addition of 100 µg/mL of equol had forms longer and thinner than those of cells in the control.

### [Example 6] Inhibition of Sporulation

To validate the influence of equol (100 µg/mL) on the sporulation of *Clostridioides difficile,* equol was added to a medium in which *Clostridioides difficile* had grown to a saturated state, and the number of spores formed was counted.

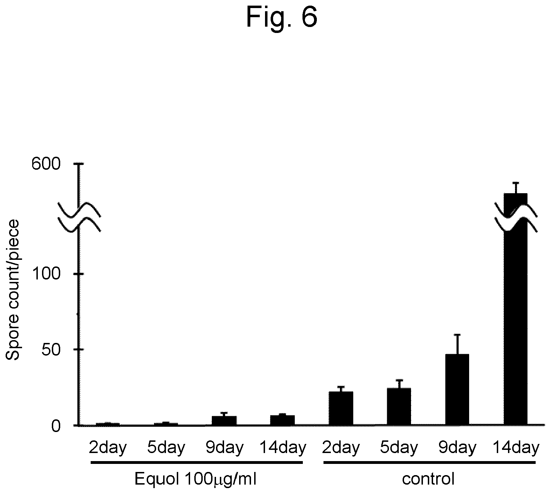

Figure 6 shows the results. As demonstrated in Figure 6, numerous spores were present in the control in every day from day 2; by contrast, the medium with addition of equol had few spores.

### Industrial Applicability

The antibacterial agent comprising equol as an active ingredient according to the present invention exhibits antibacterial action against a microorganism selected from the group consisting of *Clostridioides difficile, Clostridioides perfringens,* and *Bacteroides fragilis,* and, for example, can prevent or treat *Clostridioides difficile* infection, and can prevent nosocomial infection due to *Clostridioides difficile.*

All the publications, patents, and patent applications cited herein are directly incorporated herein by reference.

## Claims

1. An antibacterial agent against an enterobacterium, comprising equol as an active ingredient.

2. The antibacterial agent according to claim 1, wherein the enterobacterium is selected from the group consisting of *Clostridioides difficile, Clostridioides perfringens* (*C. perfringens*), and *Bacteroides fragilis* (*B. fragilis*).

3. The antibacterial agent according to claim 1 or 2, wherein the antibacterial agent inhibits the growth of *Clostridioides difficile* and inhibits the sporulation of *Clostridioides difficile.*

4. A preventive or therapeutic agent for *Clostridioides difficile* infection, comprising the antibacterial agent according to any one of claims 1 to 3.

5. A drug for controlling intestinal function comprising equol as an active ingredient.

6. The drug for controlling intestinal function according to claim 5, wherein the drug for controlling intestinal function mitigates symptoms or a symptom of diarrhea and/or abdominal pain caused by the enterobacterium.

7. A preventive and/or therapeutic agent for colorectal cancer, comprising equol as an active ingredient.
